# EUROPEAN PATENT SPECIFICATION

(11) **EP 3 367 970 B1**
(45) Date of publication and mention of the grant of the patent: **08.09.2021**
(21) Application number: 16860719.0
(22) Date of filing: 26.10.2016
(51) Int. Cl.: A61F 2/12, A61L 27/18

(54) **POST-LUMPECTOMY BREAST IMPLANT**
POST-LUMPEKTOMIE-BRUSTIMPLANTAT
IMPLANT MAMMAIRE POST-LUMPECTOMIE

(30) Priority: 30.10.2015 US 201562248906 P
(43) Date of publication of application: 05.09.2018
(73) Proprietor: Power-Cooper, Jeannette, Renton, Washington 98055 (US); Tseng, Mark, Medina, Washington 98039 (US)
(72) Inventor: Power-Cooper, Jeannette, Renton, Washington 98055 (US); Tseng, Mark, Medina, Washington 98039 (US)
(74) Representative: Grünecker Patent- und Rechtsanwälte PartG mbB
(86) International application number: PCT/US2016/058931
(87) International publication number: WO 2017/075094

(56) References cited:
- WO-A2-2011/086537
- US-A- 5 922 024
- US-A1- 2006 282 164
- US-A1- 2007 123 983
- US-A1- 2013 226 296
- US-A1- 2014 100 656
- US-A1- 2014 100 656
- US-B1- 6 387 133
- US-B2- 8 668 737

## Description

### BACKGROUND

### Technical Field

The present disclosure relates generally to breast implants, and more specifically to breast implants for post-lumpectomy implantation.

### Description of the Related Art

Breast implants are often used to reconstruct a patient's breast or breasts after surgery. For example, a diagnosed breast cancer patient can have breast tissue including cancerous tissue removed, or a patient identified as being at a high-risk for developing breast cancer can have breast tissue removed. In either case, the patient may desire reconstructive surgery. A variety of breast implants are available to such patients. A variety of breast implants are also available to patients seeking cosmetic breast augmentation.

### BRIEF SUMMARY

Provided herein is a kit of post-lumpectomy breast implants comprising multiple post-lumpectomy breast implants each including a spherical outer shell comprising a medical-grade silicone and an inner chamber, within the outer shell, filled with medical-grade silicone gel; the spherical outer shell and the inner chamber having a total volume of less than 100cc; and breast implant sizers including a device having a shape and volume matching that of each of the post-lumpectomy breast implants; wherein the total volume of each of the beast implants in the kit differs from the volume of the others by at least 5cc.

### BRIEF DESCRIPTION OF THE SEVERAL VIEWS OF THE DRAWINGS

Figure 1 illustrates a cross-sectional view of a post-lumpectomy breast implant, according to one or more illustrated embodiments.
Figure 2 illustrates a kit of post-lumpectomy breast implants, according to one or more illustrated embodiments.

### DETAILED DESCRIPTION

Various types of breast implants are currently commercially available. For example, saline breast implants include an outer silicone shell filled with sterile saline. Saline breast implants can be relatively safe because if they leak, the saline can be easily absorbed and expelled by the human body. Silicone breast implants include an outer silicone shell filled with a silicone gel. Silicone breast implants are often considered to provide a more natural feel than saline breast implants. Form-stable breast implants, sometimes referred to as "gummy bear breast implants," include an outer silicone shell filled with a thicker silicone gel than other silicone breast implants. Form-stable breast implants can be firmer than other breast implants, and can allow breast implants to be manufactured in a greater variety of specifically contoured, stable shapes. Form-stable breast implants can be less likely to break than other breast implants, and can maintain their shape even if the outer silicone shell breaks.

Surgical procedures available to breast cancer patients, patients at a high risk for developing breast cancer, or other patients include mastectomy, wherein the whole breast is removed, quadrantectomy, wherein a quarter of the breast is removed, and lumpectomy, wherein a small part of the breast is removed. As used herein, a "lumpectomy" can include the removal of any small portion of a breast, and includes a biopsy. Reconstructive surgery is often performed for patients who have undergone a mastectomy, but generally is not performed for patients who have undergone a lumpectomy. Patients who have undergone a lumpectomy often choose to live with the relatively small resulting deformations (relative to deformations resulting, e.g., from a mastectomy), rather than undergo further surgical procedure(s).

Thus, breast implants designed for reconstructive applications are typically shaped and sized to replace an entire human breast or a large portion of a human breast, rather than to replace a relatively small portion of a breast removed during a lumpectomy. Similarly, breast implants designed for cosmetic surgical applications are typically shaped and sized to uniformly increase the size of a natural human breast, rather than to occupy a relatively localized portion of a human breast. Implants of which the size can be adjusted are known. Implantation of a plurality of silicone microbeads for breast augmentation are disclosed in US2007/0123983 A1. Implantation of a inflatable implant for breast augmentation is disclosed in US2014/0100656 A1. Thus, typical breast implants now commercially available are not suitable for reconstructive use with lumpectomy patients.

Lack of options for suitable commercially available implants may deter lumpectomy patients from undergoing reconstructive surgery after their lumpectomy, and may deter physicians from recommending the same. Resulting lack of patient interest may in turn deter breast implant manufacturers from developing, producing, and marketing such implants. Whatever the reasons, the medical reality is that lumpectomy patients typically do not undergo reconstructive surgery after having their lumpectomy. Thus, this disclosure relates to post-lumpectomy breast implants for patients who have undergone a lumpectomy and desire reconstructive surgery.

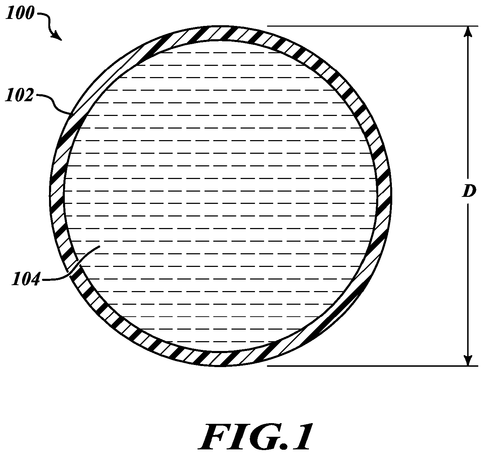

Figure 1 illustrates a cross-sectional view of one embodiment of a post-lumpectomy breast implant 100. Breast implant 100 includes an outer sac or shell 102 made of a medical grade silicone elastomer and an interior open space or chamber 104 filled with a medical grade clear silicone gel. The breast implant 100 is pre-filled, that is, the chamber 104 is filled by a manufacturer prior to the implant being shipped to a physician. In some cases, the breast implant 100 can comprise only, or consist of, the outer shell 102 and the inner chamber 104 filled with the silicone gel. The breast implant 100 can be a single-walled implant 100, and can be elastically incompressible. The breast implant 100 is spherical, i.e., the outer shell 102 is spherical and the inner chamber 104 is spherical. The breast implant 100 is also relatively small compared to many commercially available breast implants. For example, the breast implant 100 can have a volume less than 100 cubic centimeters ("cc" or "ml") or a diameter D less than about 5.75 cm. The breast implant 100 can be used in reconstructive surgeries for post-lumpectomy patients.

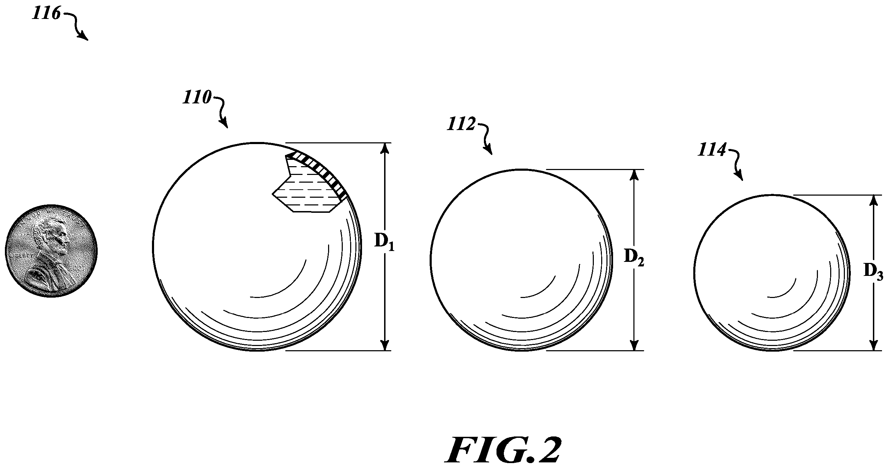

Figure 2 illustrates three different post-lumpectomy breast implants 110, 112, and 114. Breast implants 110, 112, and 114 are the same as breast implant 100 except for their volumes, and breast implant 110 is illustrated in a partial cut-away view to show that breast implant 110 can have a structure matching that of breast implant 100. Figure 2 illustrates that the breast implants described herein can have a variety of different volumes, such as volumes ranging from 25cc to 95cc. For example, the breast implants described herein can have a volume less than 100cc, 95cc, 90cc, 85cc, 80cc, 75cc, 70cc, 65cc, 60cc, 55cc, 50cc, 45cc, 40cc, 35cc, or 30cc, and/or greater than 20cc, 25cc, 30cc, 35cc, 40cc, 45cc, 50cc, 55cc, 60cc, 65cc, 70cc, 75cc, 80cc, 85cc, or 90cc. The breast implants described herein can be generally spherical and have a diameter D less than 5.75 cm, 5.50 cm, 5.25 cm, 5.00 cm, 4.75 cm, 4.50 cm, 4.25 cm, 4.00 cm, or 3.75 cm, and/or greater than 3.50 cm, 3.75 cm, 4.00 cm, 4.25 cm, 4.50 cm, 4.75 cm, 5.00 cm, 5.25 cm, or 5.50 cm. Figure 2 also illustrates a penny for a possible example of scale.

Figure 2 also illustrates a kit 116 comprising multiple breast implants 110, 112, and 114 having different volumes and hence different diameters D₁, D₂, D₃. The kit 116 can include any suitable number of breast implants, which can have any suitable volumes. For example, the kit 116 can include a plurality of breast implants each having a different volume, the volume of each breast implant in the kit differing from the volume of the others by at least 5cc, or at least 10cc, or at least 15cc. In some embodiments, the kit 116 may include a plurality of post-lumpectomy breast implants having different volumes within the range of 25cc to 95cc, or having different diameters within a range of 3.50 cm to 5.75 cm.

A method of treating a patient can include a physician meeting and consulting with the patient and determining that the patient is a suitable candidate for a lumpectomy. The method can also include performing the lumpectomy, thereby creating a cavity or pocket in one of the patient's breasts into which a post-lumpectomy breast implant can later be implanted. The method can also include a physician (either the same physician who performed the lumpectomy or a different physician) obtaining a kit of potentially suitable ("candidate") pre-filled post-lumpectomy breast implants, each having a different volume, and respective breast implant sizers. A breast implant sizer includes a device having a shape and volume matching that of a respective candidate post-lumpectomy breast implant, and can be temporarily inserted intraoperatively into the pocket in the patient's breast to evaluate the effect of the respective candidate breast implant on the patient's breast.

The method can also include the physician using one or more breast implant sizers of the kit to determine an appropriate size of a post-lumpectomy breast implant for use in reconstructive surgery of the patient's breast. The method can also include the physician making an initial estimate of a volume of the pocket or of a volume of a suitable post-lumpectomy breast implant, and selecting a corresponding breast implant sizer for evaluation. The method can also include the physician using the selected breast implant sizer to evaluate the effect of a respective candidate breast implant on the patient's breast. If the physician determines that the candidate breast implant is suitable for use, then the physician can proceed to implant the suitable candidate breast implant in the pocket in the patient's breast and complete the procedure. If the physician determines that the candidate breast implant is not suitable, e.g., that a larger or a smaller breast implant would be more suitable, then the physician can select another breast implant sizer accordingly and return to using the selected breast implant sizer to evaluate the suitability of a respective candidate breast implant. The physician can repeat this process until either a suitable post-lumpectomy breast implant is identified or the physician concludes that the patient is not suitable for reconstructive surgery with a post-lumpectomy breast implant.

Once a suitable post-lumpectomy breast implant is identified by the physician, the physician can implant the identified breast implant within the pocket in the patient's breast, and the physician can complete the operation. The method can include performing a lumpectomy and reconstructing the patient's breast using a post-lumpectomy breast implant in a single procedure, which can be performed in a single day. The post-lumpectomy breast implants described herein are fabricated from silicone materials and are therefore not bio-degradable and can prevent or resist tissue ingrowth and vascularization into the breast implant. The post-lumpectomy breast implants described herein can therefore be considered permanent and can be easily removed at a later date if removal is medically called for or otherwise desired by the patient. The post-lumpectomy breast implants described herein can also allow a physician to completely and snugly fill the pocket in the patient's breast, thereby providing pressure sufficient to prevent or reduce seroma or hematoma formation therein. The post-lumpectomy breast implants described herein can also be radiopaque, which can facilitate a physician's distinguishing between native tissue and the breast implant under x-ray.

In other embodiments, the post-lumpectomy breast implants described herein can also be used as testicular implants.

U.S. patent application Serial No. 62/248,906, filed October 30, 2015.

## Claims

1. A kit (116) of post-lumpectomy breast implants (110,112,114), comprising:
multiple post-lumpectomy breast implants (110, 112, 114) each including:
a spherical outer shell (102) comprising a medical-grade silicone; and
an inner chamber (104) within the outer shell (102) filled with a medical-grade silicone gel;
the spherical outer shell (102) and the inner chamber (104) having a total volume of less than 100cc;
and
breast implant sizers, including
a device having a shape and volume matching that of each of the post-lumpectomy breast implants (110,112,114);
wherein the total volumes of each breast implant (110,112,114) in the kit differs from the volume of the others by at least 5cc.

2. The kit of post-lumpectomy breast implants (110,112,114) of claim 1 wherein the total volumes differ from one another by at least 10cc.

3. The kit of post-lumpectomy breast implants (110,112,114) of claim 1, wherein the total volumes differ from one another by at least 15cc.

## Patentansprüche

1. Kit (116) für Brustimplantate (110, 112, 114) nach einer Lumpektomie, umfassend:
mehrere Post-Lumpektomie-Brustimplantate (110, 112, 114), die jeweils umfassen:
eine kugelförmige Außenhülle (102), umfassend ein medizinisches Silikon; und
eine Innenkammer (104) innerhalb der Außenhülle (102), die mit einem medizinischen Silikongel gefüllt ist;
wobei die kugelförmige Außenhülle (102) und die Innenkammer (104) ein Gesamtvolumen von weniger als 100 cm³ aufweisen;
und
Brustimplantat-Sizer, umfassend
eine Vorrichtung mit einer Form und einem Volumen, das jedem der Post-Lumpektomie-Brustimplantate (110,100 12,114) entspricht;
wobei sich das Gesamtvolumen jedes Brustimplantats (110,100 12,114) in dem Kit um mindestens 5 cm³ von dem Volumen der anderen unterscheiden.

2. Kit (116) für Brustimplantate (110, 112, 114) nach der Lumpektomie nach Anspruch 1, wobei sich die Gesamtvolumen um mindestens 10 cm³ voneinander unterscheiden.

3. Kit (116) für Brustimplantate (110, 112, 114) nach der Lumpektomie nach Anspruch 1, wobei sich die Gesamtvolumen um mindestens 15 cm³ voneinander unterscheiden.

## Revendications

1. Kit (116) d'implants mammaires post-lumpectomie (110, 112, 114), comprenant :
de multiples implants mammaires post-lumpectomie (110, 112, 114) incluant chacun :
une coque externe sphérique (102) comprenant un silicone de qualité médicale ; et
une chambre interne (104) à l'intérieur de la coque externe (102) remplie d'un gel de silicone de qualité médicale ;
la coque externe sphérique (102) et la chambre interne (104) ayant un volume total inférieur à 100 cc ; et
des dispositifs de dimensionnement d'implant mammaire, comprenant
un dispositif ayant une forme et un volume correspondant à celui de chacun des implants mammaires post-lumpectomie (110, 112, 114) ;
les volumes totaux de chaque implant mammaire (110, 112, 114) dans le kit différant du volume des autres d'au moins 5 cc.

2. Kit d'implants mammaires post-lumpectomie (110, 112, 114) selon la revendication 1, les volumes totaux différant l'un de l'autre d'au moins 10 cc.

3. Kit d'implants mammaires post-lumpectomie (110, 112, 114) selon la revendication 1, les volumes totaux différant l'un de l'autre d'au moins 15 cc.
